# EUROPEAN PATENT SPECIFICATION

(11) **EP 0 784 987 B1**
(45) Date of publication and mention of the grant of the patent: **01.10.2003**
(21) Application number: 97300087.0
(22) Date of filing: 08.01.1997
(51) Int. Cl.: A61L 27/16, A61L 27/18, A61L 27/20, A61L 27/52, A61F 2/12, A61F 2/26

(54) **Method of making in situ filler material for mammary, penile and testicular prosthesis and tissue expanders**
Verfahren zur Herstellung in situ von einem Füllmaterial für Brust-, Penis- und Hodenprothese, und Gewebedilatatoren
Procédé pour la fabrication in situ de matière de remplissage pour prothèse mammaire, pénienne et testiculaire et dilatateurs de tissu

(30) Priority: 16.01.1996 US 585622
(43) Date of publication of application: 23.07.1997
(73) Proprietor: MENTOR CORPORATION, Santa Barbara, CA 93111 (US)
(72) Inventor: Purkait, Bobby, Montecito, California 93108 (US)
(74) Representative: Thomson, Paul Anthony

(56) References cited:
- EP-A- 0 727 232
- EP-A- 0 730 847
- WO-A-94/25078
- FR-A- 2 691 068

## Description

### BACKGROUND OF THE INVENTION

### 1. FIELD OF THE INVENTION

The present invention relates to a prosthesis which contains a dehydrated substance that forms a gel when mixed with an aqueous solution.

### 2. DESCRIPTION OF RELATED ART

Mammary implants are typically constructed from a flexible outer shell that is filled with a fluid. A mammary prosthesis is implanted by initially creating a small incision in the breast and then inserting the flexible shell. The shell is typically in a fully deflated or semi-deflated condition when inserted into the breast. Once inserted into the mammary gland the flexible shell is filled with a fluid. The fluid can be introduced to the inner cavity of the prosthesis either through a syringe that penetrates a fill port of the outer shell, or a reservoir integral with the implant.

Mammary implants of the prior art have been filled with silicone based gels. Gels create a prosthesis which has the natural appearance and feel of a breast. When a semi-filled implant is initially inserted into the breast, the outer shell undergoes a series of stresses as the surgeon pushes and manipulates the implant into the mammary gland. The stresses may cause the outer shell to rupture, thereby exposing the patient to the silicone gel. Additionally, the shell may rupture after the prosthesis is implanted because of crease-fold flaw and/or external stresses on the breast, again allowing the inner gel to flow into the body. The recovery of gel from the body is cumbersome and difficult.

Mammary implants are now typically filled with a saline solution. Unfortunately, saline solutions do not provide the same natural appearance and feel as a gel filled implant. Additionally, saline implants are not widely used for reconstruction purposes. It is therefore desirable to provide a mammary implant that contains a gel which is biocompatible and can provide a similar "feel" as silicon gel.

U.S. Patent No. 4,731,081 issued to Tiffany et al. discloses a number of implant materials including polyvinylpyrrolidone (PVP), polyvinyl alcohol, hydroxyethyl starch, lecithin, peanut oil, cottonseed oil, fatty acid salts and fatty acid esters which have been proposed to prevent the crease-fold flaw problem associated with implanted prosthetics.

U.S. Patent No. 4,772,284 issued to Jefferies et al. discloses implant fluids which include a collagen gel and a purified gel of poly-alpha amino acid homopolymers or random copolymers having a molecular weight between 5,000 to 400,000.

U.S. Patent No. 4,787,905 issued to Loi discloses mammary prosthetic fluids that include a mixture of hydroxyl terminated polybutadiene resin, diundecyl phthulatc, polymethylene polyphenyl isocyanate and dibutyltin dilaurate catalyst which cures to form a gel.

U.S. Patent No. 5,219,360 issued to Georgiade discloses implant filling material comprising a gel made of cross-linked hyaluronic acid.

As shown in the Tiffany and Georgiade patents, the gel is injected into the prosthesis after implantation. The gel is typically provided in a separate bag that is packaged and sold with the flexible outer shell. The bag of gel is susceptible to rupture during shipping and storage, thereby creating a defective product. The prefilled product also requires special packaging and sterilization techniques to assure product sterility and storage integrity. Additionally, some countries do not allow the importation of fluid filled implants. It would therefore be desirable to provide a "dry" prosthesis product which can be filled with a gel at the surgical site.

### SUMMARY OF THE INVENTION

The present invention is an inflatable prosthesis which contains a dehydrated substance that forms a gel when mixed with an aqueous solution. The dehydrated substance is a biocompatible material such as an hydrophilic polymer which includes but is not limited to polyacrylamide, polyvinylprolidone, hydroxypropyl methylcellulose, polyvinyl alcohol, polyethylene oxides, polypropylene oxides, polyethylene glycol, polylactic, polyglycoloic acids, hydrogel polyurethane, chrondotoin sulfate, hyaluronic acid and alginate. The prosthesis includes a flexible inflatable outer shell that has an inner cavity. The inner cavity may contain the sterile dehydrated substance. The prosthesis is provided to the surgical site while the substance is in the dehydrated state. An initial volume of aqueous solution can be added to the inner cavity of the outer shell. The dehydrated substance combines with the aqueous solution to form a gel within the implant. The semi-inflated prosthesis can be implanted into a breast and inflated to a desired size with an additional volume of aqueous solution. The dehydrated substance may be coated along the inner surface of the prosthesis to form a lubricant which reduces crease-fold rupture. As an alternate embodiment, the dehydrated substance may be supplied in a package separate from the outer shell. An aqueous solution can be added to the package in situ to form a gel which can be subsequently added to the inner cavity of the outer shell.

### BRIEF DESCRIPTION OF THE DRAWINGS

The objects and advantages of the present invention will become more readily apparent to those ordinarily skilled in the art after reviewing the following detailed description and accompanying drawings, wherein:
Figure 1 is a perspective view of a prosthesis of the present invention;
Figure 2 is a cross-sectional view of the prosthesis;
Figure 3 is a perspective view showing the prosthesis being filled with an aqueous solution;
Figure 4 is a cross-sectional view showing an alternate embodiment of a prosthesis which has an inner coating of dehydrated substance;
Figure 5 is a cross-sectional view showing an implanted prosthesis which has a fold;
Figure 6 is a perspective view showing a package which has an implant and a separate package of dehydrated substance.

### DETAILED DESCRIPTION OF THE INVENTION

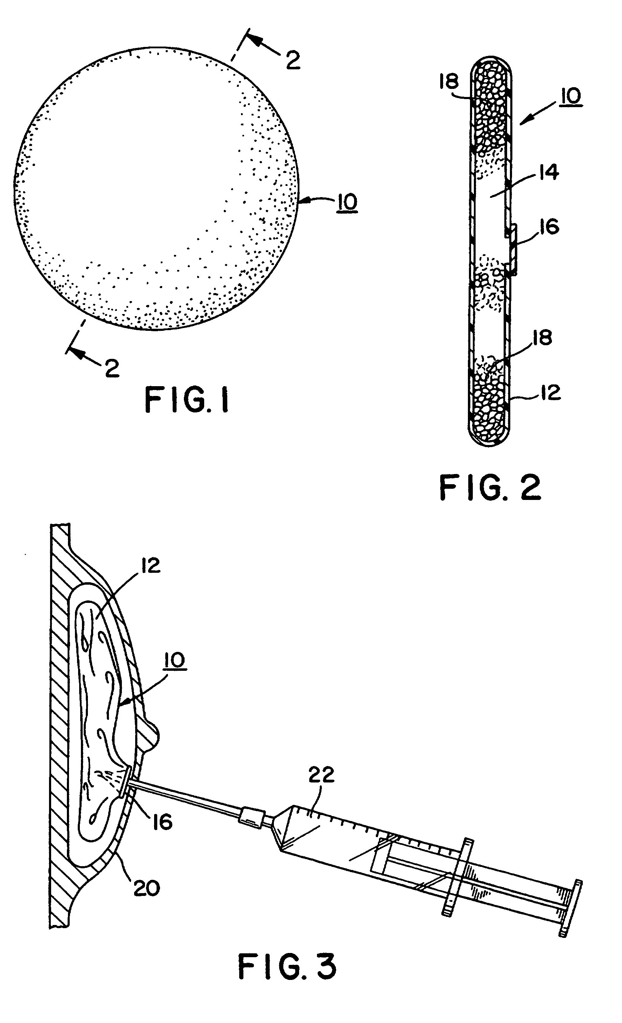

Referring to the drawings more particularly by reference numbers, Figure 1 shows a prosthesis 10 of the present invention. The prosthesis 10 may be an implant for a mammary gland. Although a mammary implant is shown and described, it is to be understood that the prosthesis 10 of the present invention may be used as a testicular implant, penal implant or a tissue expander.

As shown in Figure 2, the prosthesis 10 includes an outer shell 12 which has an inner cavity 14. The outer shell 12 is preferably constructed from a flexible inflatable material such as polydimethyl siloxane, polyurethane, polyurethane/polyester copolymer, or other similar viscoelastic material. The outer shell 12 has a fill port 16 which can receive the needle of a syringe to allow a fluid to be injected into the inner cavity 14. Alternatively, the prosthesis may have an integral reservoir (not shown) that fills the inner cavity 14 with fluid, such as the device shown and described in U.S. Patent No. 4,643,733 issued to Becker.

Located within the inner cavity 14 is a dehydrated substance 18. The dehydrated substance 18 preferably forms a gel when mixed with a fluid such as an aqueous solution. The fluid may be a saline solution. The substance 18 can be provided as a powder or in a solid form. The substance may be a hydrophilic polymer such as linear polyacrylamide, cross-linked polyacrylamide, polyvinylprolidone, hydroxypropyl methylcellulose, and its derivatives, polyvinyl alcohol and its derivatives, polyethylene oxides and its derivatives, polypropylene oxides and its derivatives, polyethylene glycol and its compounds, polylactic and polyglycolic acids and its derivatives, hydrogel polyurethane, hyaluronic acid, alginate and its derivatives, chitosan, chrondotoin sulfate and glucosamine. The substance can be added to the inner cavity 14 either before or after the outer shell 12 is sealed. Additionally, air may be evacuated from the shell 12 after the substance 18 is added to the cavity 14.

The prosthesis 10 is typically implanted into a mammary gland by initially cutting a small incision into the breast and inserting the shell 12 through the incision. A small amount of aqueous solution may be added to the prosthesis to partially fill the device before insertion into the mammary gland. A partially filled prosthesis may be easier to manipulate into the breast than an empty implant.

As shown in Figure 3, after the prosthesis 10 is implanted into the mammary gland 20, the aqueous solution may be added to the inner cavity 14 by a syringe 22. The addition of the aqueous solution combines with the dehydrated substance 18 to form a gel within the implant 10. The aqueous solution can be added until the implant is inflated to a desired volume. When the prosthesis 10 is used as a tissue expander, the aqueous solution can be subsequently added to further enlarge the device 10.

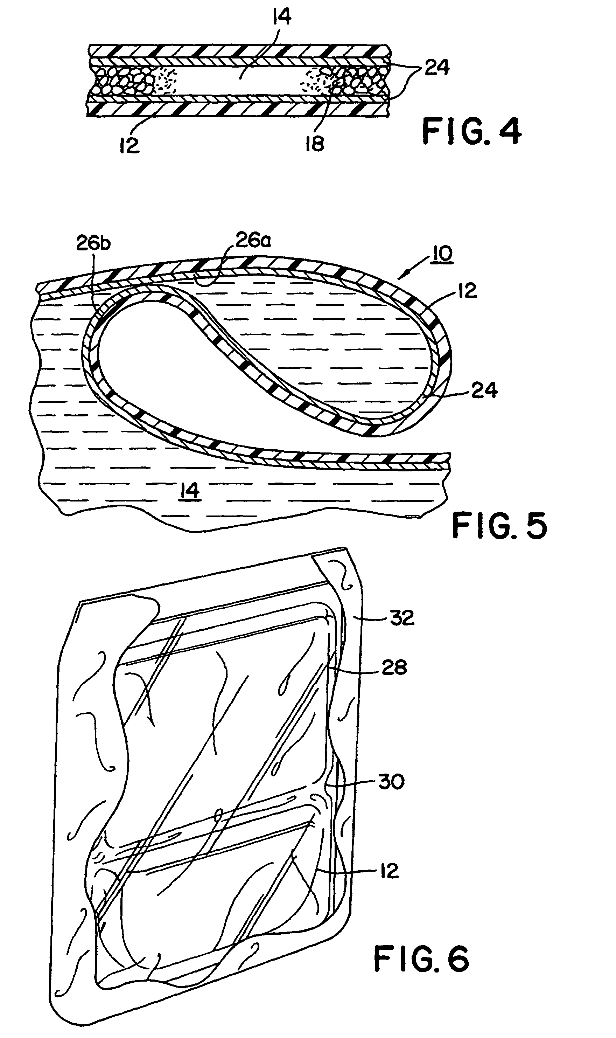

As shown in Figure 4, the prosthesis 10 may have a layer 24 of dehydrated substance that coats the inner surface of the outer shell 12. When mixed with the aqueous solution the layer of dehydrated substance forms a gel that functions as a lubricant for the inner shell surface.

As shown in Figure 5, after implantation the prosthesis 10 may fold to place two inner wall surfaces 26a and 26b of the outer shell 12 adjacent to each other. Any relative movement between the inner wall surfaces 26a and 26b may create friction and stress on the outer shell 12. The friction and stress may cause a rupture of the outer shell 12. The lubricant layer 24 reduces the friction created by the movement of the adjacent walls and decreases the likelihood of post implantation rupture of the implant. The lubricant layer 24 may be used with a gel or a non-gel fluid (purely aqueous solution) within the inner cavity 14.

As shown in Figure 6, the dehydrated substance 18 may be contained within a secondary package 28 separate from the outer shell 12. The secondary package 28 and outer shell 12 may be further packaged, shipped and stored in a sterile zip-lock bag 30. The bag 30 may be further packaged in a foil pouch 32. The package and shell are sterilized with conventional sterilization techniques including known autoclave sterilization processes.

To implant the prosthesis 10, the bag 30 may be removed from the pouch 32. Electron beam irradiation, gamma irradiation or autoclave may be used to sterilize the package 28 and the shell 12. The package 28 and shell 12 may then be removed from the bag 30. An aqueous solution may be added to the secondary package 28 to form a gel with the dehydrated substance 18. The secondary package 28 may have a fill port (not shown) to receive the needle of a syringe. The gel can then be transferred from the secondary package 28 to the inner cavity 14 of the shell 12.

Although use of a dehydrated substance 18 with an outer shell 12 has been shown and described, the substance 18 may be injected directly into body tissue in the dehydrated form. The fluids and temperature of the body may then form the substance into a gel. When injected into a mammary gland the dehydrated substance will become soft, pliable, bulky and function as breast tissue.

Listed below are preferred compositions and methods for creating exemplary gels within the package 28 or shell 12.

### Alginate

A calcium alginate can be made by combining sodium alginate with calcium chloride or other calcium salts in an appropriate ratio. The composition may consist of the following components:
Sodium alginate: 0.25% to 15% and preferably, 1 to 5%, and most preferably, 2%
Calcium chloride dehydrate (CaCl₂•2H₂O): 0.1% to 1.2%, and preferably 0.2% to 0.5%, and most preferably 0.32%.
PC (phosphatidylcholine): 0.001% to 0.5%, preferably 0.005% to 0.15%, and most preferably 0.01%.
Sodium Chloride: 0.2% to 0.5% to make the formulation isotonic.
Ethanol: 0.1% to 8%, preferably 0.5% to 5%.

When mixed with an aqueous solution, the osmolity range for the final alginate gel may be 200 mOsm/kg to 400 mOsm/kg, and preferably 250 mOsm/kg to 350 mOsm/kg. The viscosity of the alginate gel may be in the range of 5 Nm⁻²s to 150 Nm⁻²s (5,000 cP to 150,000 cP), and preferably 50 Nm⁻²s to 1200 Nm⁻²s (50,000 cP to 120,000 cP)as measured by a Brookfield model DV-II+ viscometer at and a shear rate of 0.42sec⁻¹. The pH of the final alginate gel may be broadly in the range of 5 to 10, and preferably 6 to 8. The alginate may have a guluronic acid content above 30%. Also, bacterial alginate which has been modified with a mannuronic acid epimerase would fulfill the necessary characteristics of an appropriate alginate material.

Phosphatidylcholine (PC) is a desired component of the final gel. It provides several different functions. PC is amphiphilic, thus, it is a lubricity agent for the shell. PC also competes with the alginate chains for calcium cations, transforming a calcium alginate brittle gel into a flowing gel. This property is consistent with previous uses of PC as a gel dispersant and is believed to aid in excretion of large quantities of the gel should the shell rupture. PC acts as an emulsifier, which has been shown to increase both polysaccharide and cephalosporin stability. The compatibility of alginate with PC has been established in other systems such as microencapsulated liposomes, wound dressings and transdermal controlled release systems.

PC has been used clinically in parenteral preparations of doxorubicin, amphotericin, benzodiazepines, penicillin, and vitamins as well as an emulsifier for essential fatty acids and oils in total parenteral nutrition.

It may also be desirable to incorporate antimicrobial and antifungal agents into the final gel of the prosthesis. Although alginates are very resistant to bacteria when compared to other polysaccharides, some microorganisms do produce alginases. Bacterial and fungal growth studies were performed using the most commonly encountered bacteria and fungi in the operating rooms of hospitals. The study concluded that 0.06% ceftazidime and 0.008% miconazole produced a three (3) or more log reduction in the population of all the challenging organisms. Miconazole is a broad spectrum antifungal, that also has activity against gram-positive bacteria. In addition, the mentioned concentration of ceftazidime is higher than the minimal inhibitory concentration of all reported ceftazidime resistant strains; however, should simultaneous rupture of 2-800 cc implants occur, the amount of ceftazidime and miconazole released would be lower than a single prophylactic intravenous or intramuscular dose, and therefore, the amounts of antibiotics used in the present invention are believed to be safe.

A polar steroid such as cortisol has a permeation value for silicone rubber of 0.00025 µg mm⁻¹h⁻¹. PC, miconazole and ceftazidime may be chosen with polar groups and molecular weights greater than steroids (330-450Da.), so that migration through the shell should be negligible.

### Sodium Hyaluronate

1. 1 to 10% hyaluronic acid with modified balanced salts (USP or NF grade): 0.075% KCl, 0.048% CaCl₂, 0.030% MgCl₂, and NaCl in sterile pyrogen-free water to achieve the osmolality range specified below. (MgCl₂ also serves as an antioxidant agent.)
2. 0.03 to 0.13% Ceftazadime and 0.002 to 0.05% miconazole.
3. The viscosity of the hyaluronic acid gel is 5 Nm⁻²s to 150 Nm⁻²s (5,000 - 150,000 cps) at 37°C and the osmolality is 200 to 400, and preferably 250-350 mOsm/kg.

### PVA

3A. 6% polyvinyl alcohol (PVA) (M.W. equivalent to Elvanol 52-22; 40 KD) with 0.07 ml of 25% glutaraldehyde (cross-linking agent), 0.3 ml 85% H₃PO₄, 4 ml 1.85 M NaOH per 100 ml of 6% PVA solution and 0.66% NaCl in sterile pyrogen-free water.
   or
3B. 3% PVA (M.W. equivalent to Elvanol 50-42; 75 KD) with 0.06 ml of 25% glutaraldehyde (cross-linking agent), 0.3 ml 85% H₃PO₄, 4 ml 1.85 M NaOH per 100 ml of 6% PVA solution and 0.66% NaCl in sterile pyrogen-free water.
   or
3C. 10% PVA (M.W. equivalent to Elvanol 52-22; 40 KD) and 0.66% NaCl in sterile pyrogen-free water buffered to pH 7 with 0.05M NaH₂PO₄.
   or
3D. 2 to 30% PVA synthesized from Ethanol based Solvent (MW range 10 - 50 KD) with similar combination of crosslinking agent, buffering and stability agents as previously discussed in 3A, 3B or 3C.

The molecular weight which provides an appropriate viscosity for PVA is preferably in the range of 10,000 to 40,000 Daltons.

### HPMC

Hydroxypropylmethyl cellulose (HPMC) and its derivatives may be employed as filling materials in the present invention. Derivatives of HPMC include hydroxyalkyl cellulose, sodium carboxymethyl cellulose, hydroxyethyl cellulose, hydroxypropyl cellulose, methylhydroxypropyl cellulose, methyl cellulose and ethylhydroxyethyl cellulose.

1 to 10% HPMC solid content of low to medium molecular weight (MW 10,000 to 40,000 Dalton). The viscosity can be enhanced with the addition of less than 1% p-benzoquinine. Similar antimicrobial and antifungal agents as those discussed in the above examples are used.

### POLYACRYLAMIDE

Polyacrylamide, in a linear, branched chain, or cross-linked form can be used as a filling material in accordance with the present invention. As a preliminary matter, it has been found that commercially available material is not sufficiently pure, and therefore, purification is desired for the preferred embodiment. The following provides a process for purifying polyacrylamide.

### Raw Materials:

Acrylamide, Electrophoresis Grade or other commercially available
N, N'-methylenebisacrylamide
Triethanol amine (TEA)
Ammonium Persulfate
Ammonium Sulfate
Deionized Water (DI water), 12 MΩ or better
Ethanol, USP

### Polymerization

The polymerization is carried out in solution. The concentration is selected so that the resulting polymer solution is stirrable.

Acrylamide (and methylenebisacrylamide, or other crosslinking agent, if used) and ammonium sulfate are dissolved in DI water in a polymerization vessel. While stirring, the solution is charged with nitrogen and the vessel continuously purged with nitrogen to eliminate and exclude oxygen from the reaction. Ammonium persulfate and TEA are dissolved in DI water in separate containers and the solutions sparged with nitrogen. The reaction is initiated by adding the ammonium persulfate solution followed by the TEA solution without admitting oxygen to the reactor. The reaction may be run adiabatically or the temperature controlled by a heating or cooling bath. The polymerization is run essentially to completion, which typically requires several hours.

### Precipitation

The polymer is separated from the solution by precipitation with alcohol. Any lower alcohol may be used, but preferably ethanol is used. The precipitation may be carried out in the polymerization reactor, or the polymer solution transferred to a suitable vessel.

While stirring the solution, alcohol is added slowly until the polymer becomes insoluble and precipitates from solution. This occurs rapidly over a very narrow range of alcohol concentration. More alcohol is added to bring the slurry volume to about four times the initial solution volume and stirring continued for a period of time to allow the polymer particles to equilibrate with the alcohol water solution and firm up. The solid polymer is then collected on a filter. The polymer particles are then reslurried in alcohol and stirred for a period of time. The collection and reslurring is repeated. At this point, the polymer has previously been dried, but it would be more efficient to hold it as a slurry in alcohol.

### Purification

Most of the salts and unreacted monomer remained in the aqueous phase on precipitation of the polymer. The residual monomer is reduced to an acceptable level by extraction with water-alcohol solution.

Dry polymer powder or polymer filtered from alcohol-water slurry is placed into a beaker or other suitable vessel and slurried in alcohol water solution. The alcohol concentration is adjusted so that the polymer particles will be swollen, but will not agglomerate. After a period of time, the alcohol concentration is increased to firm up the particles for separation from the liquid on a filter. This process is repeated for four extraction cycles. The polymer particles are then slurried in an alcohol-water solution with the alcohol concentration adjusted so as to produce a desirable residual alcohol content in the dried polymer. The polymer is collected on a filter and dried.

### Drying

The mass of wet polymer is spread on glass trays and vacuum dried without heat. This typically requires two days. For larger volumes, a vacuum tumble dryer would be effective.

While certain exemplary embodiments have been described and shown in the accompanying drawings, it is to be understood that such embodiments are merely illustrative of and not restrictive on the broad invention, and that this invention not be limited to the specific constructions and arrangements shown and described, since various other modifications may occur to those ordinarily skilled in the art.

## Claims

1. A prosthesis (10) that can be filled with a fluid, comprising:
an outer shell (12) having an inner cavity (14); and,
dehydrated substance (18; 24) within said inner cavity (14),
**characterised in that** said dehydrated substance (18; 24) forms a gel when combined with the fluid.

2. A prosthesis as claimed in claim 1, **characterised in that** said dehydrated substance is biocompatible.

3. A prosthesis as claimed in claim 2, **characterised in that** said dehydrated substance is a hydrophilic polymer.

4. A prosthesis as claimed in claim 1, 2 or 3, **characterised in that** said dehydrated substance (24) is coated along an inner surface of said outer shell (12).

5. A prosthesis as claimed in any preceding claim, **characterised in that** the dehydrated substance comprises a malarial selected from the group consisting of:
linear polyacrylamide, cross-linked polyacrytamide, polyvinylpyrrolidone hydroxypropyl methylcellulose, polypropylene oxides, derivatives of polypropylene oxide, polyethylene glycol; polylactic acid, derivatives of polylactic acid, polyglycolic acid, derivatives of polyglycolic acid, hydrogel polyurethane, hyaluronic acid, alginate, derivatives of alginate, chitosan, chondroitinsulfate and glucosamine.

6. A prosthesis as claimed in any preceding claim, **characterised in that** said fluid comprises saline.

7. A prosthesis as claimed in any preceding claim, **characterised in that** said outer shell (12) is provided with a fill port (16).

8. A prosthesis as claimed in any precoding claim, **characterised in that** said outer shell (12) comprises polydimethyl siloxane, polyurethane, or a polyurethane/polyester copolymer.

9. A prosthetic assembly that can be filled with a fluid, comprising:
an outer shell (12) having an inner cavity (14);
a secondary package (28) that contains dehydrated substance (18; 24) that forms a gel when combined with the fluid and,
an outer package (30) that encloses said outer shell (12) and said secondary package (28) in a sterile condition.

10. An assembly as claimed in claim 9, **characterised in that** said dehydrated substance is biocompatible.

11. An assembly as claimed in claim 10, **characterised in that** said dehydrated substance is a hydrophilic polymer.

12. An assembly as claimed in claim 9, 10 or 11, **characterised in that** said outer shell (12), said secondary package (28) and said outer package (30) are sterilized.

13. An assembly as claimed in any one of claims 9 to 12, **characterised in that** said dehydrated substance comprises:
linear polyacrylamide, cross-linked polyacrylamide, polyvinylpyrrolidone, hydroxypropyl methylcellulose, polypropylene oxides, derivatives of polypropylene oxide, polyethylene glycol; polylactic acid, derivatives of polylactic acid, polyglycolic acid, derivatives of polyglycolic acid, hydrogel polyurethane, hyaluronic acid, alginate, derivatives of alginate, chitosan, chondroitinsulfate and glucosamine.

14. An assembly as claimed in any one of claims 9 to 13, **characterised in that** said fluid comprises saline.

15. An assembly as claimed in any one of claims 9 to 14, **characterised in that** said outer shell (12) is provided with a fill port (16).

16. An assembly as claimed in any one of claims 9 to 15, **characterised in that** said outer shell (12) comprises polydimethyl siloxane, polyurethane, or a polyurethane/polyester copolymer.

## Patentansprüche

1. Prothese (10), die mit einer Flüssigkeit gefüllt werden kann, umfassend:
eine äußere Schale (12) mit einem inneren Hohlraum (14) und in diesem inneren Hohlraum (14) eine dehydratisierte Substanz (18; 24),
**dadurch gekennzeichnet, dass** die dehydratisierte Substanz (18; 24) in Verbindung mit der Flüssigkeit ein Gel bildet.

2. Prothese nach Anspruch 1, **dadurch gekennzeichnet, dass** die dehydratisierte Substanz biokompatibel ist.

3. Prothese nach Anspruch 2, **dadurch gekennzeichnet, dass** die dehydratisierte Substanz ein hydrophiles Polymer ist.

4. Prothese nach Anspruch 1, 2 oder 3, **dadurch gekennzeichnet, dass** die dehydratisierte Substanz (24) eine Innenfläche der äußeren Schale (12) überzieht.

5. Prothese nach einem der vorstehenden Ansprüche, **dadurch gekennzeichnet, dass** die dehydratisierte Substanz einen Stoff umfasst, der ausgewählt ist aus der Gruppe bestehend aus:
linearem Polyacrylamid, vernetztem Polyacrylamid, Polyvinylpyrrolidon, Hydroxypropylmethylcellulose, Polypropylenoxiden und deren Derivaten, Polyethylenglycol, Polymilchsäure und deren Derivaten, Polyglykolsäure und deren Derivaten, Hydrogel-Polyurethan, Hyaluronsäure, Alginaten und deren Derivaten, Chitosan, Chondroitinsulfat und Glucosamin.

6. Prothese nach einem der vorstehenden Ansprüche, **dadurch gekennzeichnet, dass** die Flüssigkeit Kochsalz enthält.

7. Prothese nach einem der vorstehenden Ansprüche, **dadurch gekennzeichnet, dass** die äußere Schale (12) mit einer Füllöffnung (16) versehen ist.

8. Prothese nach einem der vorstehenden Ansprüche, **dadurch gekennzeichnet, dass** die äußere Schale (12) Polydimethylsiloxan, Polyurethan oder ein Polyurethan/Polyester-Copolymer umfasst.

9. Prothesenaufbau, der mit einer Flüssigkeit gefüllt werden kann, umfassend: eine äußere Schale (12) mit einem inneren Hohlraum (14), eine zusätzliche Packung (28), die eine dehydratisierte Substanz (18; 24) enthält, welche in Verbindung mit der Flüssigkeit ein Gel bildet, und eine äußere Packung (30), die die äußere Schale (12) und die zusätzliche Packung (28) steril umgibt.

10. Aufbau nach Anspruch 9, **dadurch gekennzeichnet, dass** die dehydratisierte Substanz biokompatibel ist.

11. Aufbau nach Anspruch 10, **dadurch gekennzeichnet, dass** die dehydratisierte Substanz ein hydrophiles Polymer ist.

12. Aufbau nach Anspruch 9, 10 oder 11, **dadurch gekennzeichnet, dass** die äußere Schale (12), die zusätzliche Packung (28) und die äußere Packung (30) sterilisiert sind.

13. Aufbau nach einem der Ansprüche 9 bis 12, **dadurch gekennzeichnet, dass** die dehydratisierte Substanz lineares Polyacrylamid, vernetztes Polyacrylamid, Polyvinylpyrrolidon, Hydroxypropylmethylcellulose, Polypropylenoxide und deren Derivate, Polyethylenglycol, Polymilchsäure und deren Derivate, Polyglykolsäure und deren Derivate, Hydrogel-Polyurethan, Hyaluronsäure, Alginate und deren Derivate, Chitosan, Chondroitinsulfat und Glucosamin umfasst.

14. Aufbau nach einem der Ansprüche 9 bis 13, **dadurch gekennzeichnet, dass** die Flüssigkeit Kochsalz enthält.

15. Aufbau nach einem der Ansprüche 9 bis 14, **dadurch gekennzeichnet, dass** die äußere Schale (12) mit einer Füllöffnung (16) versehen ist.

16. Aufbau nach einem der Ansprüche 9 bis 15, **dadurch gekennzeichnet, dass** die äußere Schale (12) Polydimethylsiloxan, Polyurethan oder ein Polyurethan/Polyester-Copolymer umfasst.

## Revendications

1. Prothèse (10) pouvant être remplie d'un fluide, comprenant :
une enveloppe extérieure (12) ayant une cavité intérieure (14) ; et
une substance déshydratée (18 ; 24) à l'intérieur de ladite cavité intérieure (14), **caractérisée en ce que** ladite substance déshydratée (18 ; 24) forme un gel lorsqu'elle est combinée au fluide.

2. Prothèse selon la revendication 1, **caractérisée en ce que** ladite substance déshydratée est biocompatible.

3. Prothèse selon la revendication 2, **caractérisée en ce que** ladite substance déshydratée est un polymère hydrophile.

4. Prothèse selon la revendication 1, 2 ou 3, **caractérisée en ce que** ladite substance déshydratée (24) est appliquée suivant une surface intérieure de ladite enveloppe extérieure (12).

5. Prothèse selon l'une quelconque des revendications précédentes, **caractérisée en ce que** la substance déshydratée comprend un matériau sélectionné dans le groupe composé de :
polyacrylamide linéaire, polyacrylamide réticulé, polyvinylpyrrolidone, hydroxypropyl méthylcellulose, oxydes de polypropylène, dérivés d'oxyde de polypropylène, potyéthylènegtycol ; acide polylactique, dérivés d'acide polylactique, acide polyglycolique, dérivés d'acide polyglycolique, polyuréthanne hydrogel, acide hyaluronique, alginate, dérivés d'alginate, chitosane, sulfate de chondroitine et glucosamine.

6. Prothèse selon l'une quelconque des revendications précédentes, **caractérisée en ce que** ledit fluide comprend un sérum physiologique.

7. Prothèse selon l'une quelconque des revendications précédentes, **caractérisée en ce que** ladite enveloppe extérieure (12) est munie d'un orifice de remplissage (16).

8. Prothèse selon l'une quelconque des revendications précédentes, **caractérisée en ce que** ladite enveloppe extérieure (12) comprend du polydiméthylsiloxane, du polyuréthanne, ou un copolymère polyuréthanne/polyester.

9. Ensemble prothétique pouvant être rempli d'un fluide, comprenant :
une enveloppe extérieure (12) ayant une cavité intérieure (14) ;
un emballage secondaire (28) contenant une substance déshydratée (18 ; 24) qui forme un gel lorsqu'elle est combinée au fluide, et
un emballage extérieur (30) qui entoure ladite enveloppe extérieure (12) et ledit emballage secondaire (28) dans un état stérile.

10. Ensemble selon la revendication 9, **caractérisé en ce que** ladite substance déshydratée est biocompatible.

11. Ensemble selon la revendication 10, **caractérisé en ce que** ladite substance déshydratée est un polymère hydrophile.

12. Ensemble selon la revendication 9, 10 ou 11, **caractérisé en ce que** ladite enveloppe extérieure (12), ledit emballage secondaire (28) et ledit emballage extérieur (30) sont stérilisés.

13. Ensemble selon l'une quelconque des revendications 9 à 12, **caractérisé en ce que** ladite substance déshydratée comprend :
polyacrylamide linéaire, polyacrylamide réticulé, polyvinylpyrrolidone, hydroxypropyl méthyloellulose, oxydes de polypropylène, dérivés d'oxyde de polypropylène, polyéthylèneglycol ; acide polylactique, dérivés d'acide polylactique, acide polyglycolique, dérivés d'acide polyglycollque, polyuréthanne hydrogel, acide hyaluronique, alginate, dérivés d'alginate, chitosane, sulfate de chondroitine et glucosamine.

14. Ensemble selon l'une quelconque des revendications 9 à 13, **caractérisé en ce que** ledit fluide comprend un sérum physiologique.

15. Ensemble selon l'une quelconque des revendications 9 à 14, **caractérisé en ce que** ladite enveloppe extérieure (12) est munie d'un orifice de remplissage (16).

16. Ensemble selon l'une quelconque des revendications 9 à 15, **caractérisé en ce que** ladite enveloppe extérieure (12) comprend du polydiméthylsiloxane, du polyuréthanne, ou un copolymère polyuréthanne/polyester.
